# EUROPEAN PATENT APPLICATION

(11) **EP 4 656 623 A1**
(43) Date of publication of application: **03.12.2025**
(21) Application number: 24822289.5
(22) Date of filing: 04.03.2024
(51) Int. Cl.: C07C 25/13, C07C 25/00, C07C 25/02, H10K 30/88, H10K 85/50, H10K 85/00, C07D 333/10, C07D 315/00

(54) **AMPHIPATHIC MOLECULE, AND PREPARATION METHOD THEREFOR AND USE THEREOF**

(30) Priority: 15.06.2023 CN 202310714608
(71) Applicant: Contemporary Amperex Future Energy Research Institute (Shanghai) Limited, Shanghai 200241 (CN); Contemporary Amperex Technology Co., Limited, Ningde, Fujian 352100 (CN)
(72) Inventor: LIU, Tianyu, Shanghai 200241 (CN); GU, Shuai, Shanghai 200241 (CN); JIA, Boyu, Shanghai 200241 (CN); GUO, Yongsheng, Shanghai 200241 (CN); CHEN, Guodong, Shanghai 200241 (CN)
(74) Representative: Lorenz Seidler Gossel Part. mbB
(86) International application number: PCT/CN2024/079780
(87) International publication number: WO 2024/255331

(57) **Abstract**

This application discloses an ambipolar molecule, a preparation method thereof, and an application thereof. The chemical structure general formula of the ambipolar molecule provided by this application is represented by formula I: 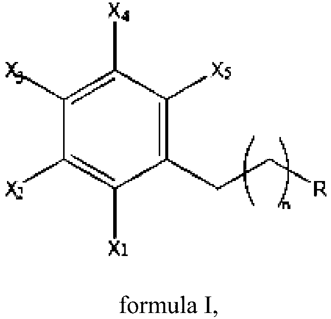 where R includes a Lewis base group, X₁, X₂, X₃, X₄, and X₅ each include at least one of a hydrogen atom and a halogen atom, X₁, X₂, X₃, X₄, and X₅ are not simultaneously hydrogen, and n is an integer greater than or equal to 1. The ambipolar molecule of this application simultaneously possesses a Lewis acid group and a Lewis base group, enabling it to passivate two types of defects at perovskite grain boundaries, namely undercoordinated anions and undercoordinated cations. Thus, when used in perovskite materials, such an ambipolar molecule can significantly improve the conversion efficiency and stability of perovskite.

## Description

This application claims priority to Chinese Patent Application No. 202310714608.X, filed with the China National Intellectual Property Administration on June 15, 2023 and entitled "AMBIPOLAR MOLECULE, PREPARATION METHOD THEREOF, AND APPLICATION THEREOF", which is incorporated herein by reference in its entirety.

### TECHNICAL FIELD

This application pertains to the technical field of perovskite technology, specifically relating to an ambipolar molecule, a preparation method thereof, and an application thereof.

### BACKGROUND

Perovskite (perovskite) materials are a class of semiconductor nanomaterials characterized by uniform size and high color purity, with a crystal structure similar to that of the mineral CaTiO₃. Perovskite materials exhibit strong light absorption capabilities and a wide absorption range, offering significant advantages in the optoelectronic field. For instance, perovskite solar cells (Perovskite solar cells, PSCs) with organic-inorganic hybrid perovskite materials as the light-absorbing layer have developed rapidly.

In an ideal perovskite crystal structure, each atom occupies its designated position. However, actual perovskite crystals are affected by crystal growth and subsequent processing, resulting in surface defects. Grain boundary defect states formed in perovskite crystals readily lead to non-radiative recombination of charge carriers, thereby affecting their photoelectric conversion efficiency.

### SUMMARY

In view of the above issues, this application provides an ambipolar molecule, a preparation method thereof, and an application thereof, aimed at addressing the technical problem of reducing perovskite grain boundary defects.

According to a first aspect, an embodiment of this application provides an ambipolar molecule, where a chemical structure general formula of the ambipolar molecule is represented by formula I: 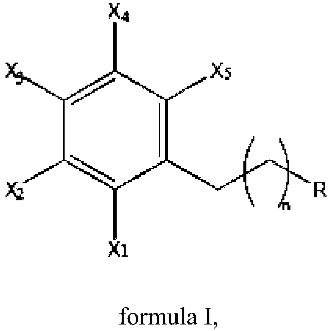 where R includes a Lewis base group, X₁, X₂, X₃, X₄, and X₅ each include at least one of a hydrogen atom and a halogen atom, X₁, X₂, X₃, X₄, and X₅ are not simultaneously hydrogen, and n is an integer greater than or equal to 1.

The ambipolar molecule simultaneously possesses a Lewis acid (Lewis Acid, LA) group and a Lewis base (Lewis Base, LB) group. Specifically, in the ambipolar molecule represented by formula I, R includes a Lewis base group, while the halogen-substituted benzene ring structure serves as a Lewis acid group. Such an ambipolar molecule can be configured to passivate two types of defects at perovskite grain boundaries, namely undercoordinated anions and undercoordinated cations. On one hand, the halogen-substituted benzene ring structure, as a Lewis acid group, can accept electrons from anions at the perovskite crystal edges to passivate undercoordinated anions in the perovskite crystal. On the other hand, the lone electron pair of the Lewis base at the R end can be provided to undercoordinated cations to passivate anion vacancy defects in the perovskite crystal. Additionally, based on the hydrophobicity of the ambipolar molecule itself, the moisture resistance of the perovskite can be improved. Therefore, the ambipolar molecule provided by the embodiments of this application can effectively passivate surface defects of perovskite crystals, thereby improving the conversion efficiency and stability of perovskite.

In an embodiment, n is 1 to 9.

The distance between the R group and the halogen-substituted benzene ring structure is relatively short, making it less likely to form intermolecular adducts. The ambipolar molecule can flexibly bind with undercoordinated anions and undercoordinated cations as needed.

In an embodiment, the Lewis base group includes at least one of thiophenyl, furanyl, pyridyl, and 3-methylimidazolyl.

The above Lewis base groups can bind with undercoordinated cations, providing lone electron pairs to passivate anion vacancy defects in the perovskite crystal.

In an embodiment, X₁, X₂, X₃, X4, and X5 are a same halogen atom.

The fully halogen-substituted benzene ring structure, as a Lewis acid group, can better bind with undercoordinated anions to more effectively passivate undercoordinated anions in the perovskite crystal.

In an embodiment, the ambipolar molecule includes:

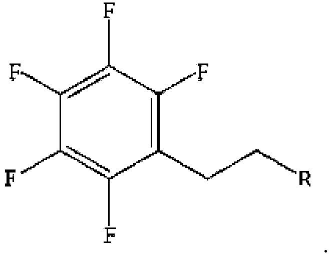

In the ambipolar molecule, the fully fluorine-substituted benzene ring serves as the Lewis acid group, which not only has a stable structure and effectively passivates perovskite grain boundary defects but is also easy to synthesize.

According to a second aspect, an embodiment of this application provides a preparation method of an ambipolar molecule, including the following step:
subjecting a compound represented by formula II to a hydrogenation reaction to obtain the ambipolar molecule provided by the first aspect of the embodiments of this application:

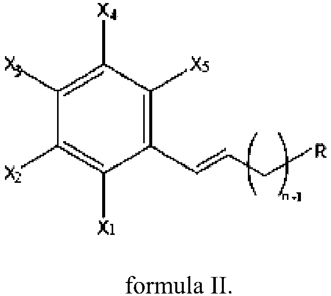

Using the compound represented by formula II for a hydrogenation reaction can yield the ambipolar molecule represented by formula I in the embodiments of this application. This preparation method is not only simple and easy to implement but also produces an ambipolar molecule with Lewis acid and Lewis base groups that effectively passivates perovskite grain boundary defects, thereby improving the conversion efficiency and stability of perovskite.

In an embodiment, a temperature of the hydrogenation reaction is 60°C to 80°C; and/or,
a duration of the hydrogenation reaction is 2 h to 8 h.

The above temperature and duration conditions effectively facilitate the synthesis of the ambipolar molecule.

In an embodiment, the hydrogenation reaction is conducted under a condition of a rhodium-based catalyst; and/or,
the hydrogenation reaction is conducted in an alcohol-based solvent.

The addition of the above catalyst effectively enhances the synthesis efficiency of the ambipolar molecule. Meanwhile, alcohol-based solvents provide a favorable environment for the synthesis of the ambipolar molecule.

In an embodiment, the synthesis steps of the compound represented by formula II include: subjecting a compound represented by formula III to a nucleophilic substitution reaction with triethyl phosphite, then subjecting the product of the nucleophilic substitution reaction to an addition reaction with R-(CH₂)ₙ₋₁-CO: 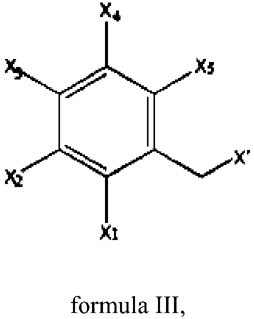 where X' is a halogen atom.

Triethyl phosphite first undergoes a nucleophilic substitution reaction with the halogenated benzene ring hydrocarbon represented by formula III, followed by a reaction with an aldehyde to yield the HWE (Horner-Wadsworth-Emmons) reaction product, which is the compound represented by formula II.

According to a third aspect, an embodiment of this application provides an application, namely the application of the ambipolar molecule provided by the first aspect of the embodiments of this application and/or the ambipolar molecule prepared by the preparation method provided by the second aspect of the embodiments of this application as a perovskite crystal passivator.

The ambipolar molecule represented by formula I, which simultaneously possesses Lewis acid and Lewis base groups, can be configured to passivate undercoordinated anions and undercoordinated cations at perovskite grain boundaries. Therefore, it can be used as a perovskite crystal passivator to passivate perovskite grain boundary defects, thereby improving the conversion efficiency and stability of perovskite.

According to a fourth aspect, an embodiment of this application provides a perovskite material, where the perovskite material includes a perovskite crystal and the ambipolar molecule provided by the first aspect of the embodiments of this application and/or the ambipolar molecule prepared by the preparation method provided by the second aspect of the embodiments of this application, bound to the perovskite crystal.

The ambipolar molecule, which simultaneously possesses Lewis acid and Lewis base groups and exhibits hydrophobicity, is used in the perovskite material as a passivator bound to the perovskite crystal, thereby passivating perovskite grain boundary defects and improving the conversion efficiency and stability of the perovskite material.

In an embodiment, a molar ratio of the perovskite crystal to the ambipolar molecule is 1:(0.0001 to 0.002).

The ambipolar molecule at the above ratio effectively passivates the perovskite crystal.

According to a fifth aspect, an embodiment of this application provides an optoelectronic device including a perovskite layer, where the perovskite layer includes the perovskite material provided by the fourth aspect of the embodiments of this application.

Since the perovskite layer of the optoelectronic device provided by the embodiments of this application includes a unique perovskite material, such a perovskite layer has fewer grain boundary defects and good moisture resistance, thereby enabling the optoelectronic device to exhibit excellent photoelectric conversion efficiency and stability.

In an embodiment, the optoelectronic device includes a solar cell.

The solar cell uses the unique perovskite material of the embodiments of this application, enabling better conversion of light energy into electrical energy, resulting in excellent photoelectric conversion efficiency and stability.

According to a sixth aspect, an embodiment of this application provides an electric apparatus including the optoelectronic device provided by the fifth aspect of the embodiments of this application.

By adopting the optoelectronic device provided by the fifth aspect of the embodiments of this application, such an electric apparatus exhibits high photoelectric conversion efficiency and good stability, enabling better operation.

The above description is merely an overview of the technical solutions of this application. To enable a clearer understanding of the technical means of this application, implementation can be carried out in accordance with the content of the specification. To make the above and other purposes, features, and advantages of this application more apparent and understandable, specific embodiments of this application are provided below.

### BRIEF DESCRIPTION OF DRAWINGS

By reading the detailed description of the preferred embodiments below, various other advantages and benefits will become clear to those of ordinary skill in the art. The drawings are provided solely for the purpose of illustrating the preferred embodiments and are not considered to limit this application. Moreover, throughout the drawings, identical components are denoted by identical reference numerals. In the accompanying drawings:
FIG. 1 is a schematic diagram comparing a perovskite before and after passivation with an ambipolar molecule according to an embodiment of this application; and
FIG. 2 is a schematic structural diagram of a solar cell according to an embodiment of this application.

Description of reference numerals:
1-first electrode, 2-hole transport layer, 3-perovskite layer, 4-electron transport layer, and 5-second electrode.

### DESCRIPTION OF EMBODIMENTS

The embodiments of the technical solutions of this application will be described in detail below with reference to the accompanying drawings. The following embodiments are merely intended for a clearer description of the technical solutions of this application and are used as examples only, which do not constitute limitations on the protection scope of this application.

Unless otherwise defined, all technical and scientific terms used herein have the same meaning as commonly understood by those skilled in the technical field of this application; the terms used herein are for the purpose of describing specific embodiments only and are not intended to limit this application; the terms "include" and "have" and any variations thereof in the specification, claims, and the above description of the drawings of this application are intended to cover non-exclusive inclusion.

In the description of the embodiments of this application, the technical terms "first", "second", and the like are used only to distinguish different objects and should not be understood as indicating or implying relative importance or implicitly indicating the number, specific order, or primary-secondary relationship of the indicated technical features. In the description of the embodiments of this application, "multiple" means two or more, unless otherwise explicitly and specifically limited.

In this specification, reference to "embodiment" means that specific features, structures, or characteristics described with reference to the embodiment may be incorporated in at least one embodiment of this application. The appearance of this phrase in various places in the specification does not necessarily refer to the same embodiment, nor is it an independent or alternative embodiment mutually exclusive with other embodiments. Persons skilled in the art explicitly and implicitly understand that the embodiments described herein may be combined with other embodiments.

In the description of the embodiments of this application, the term "and/or" is only an associative relationship for describing associated objects, indicating that three relationships may be present. For example, A and/or B may indicate the following three cases: presence of only A, presence of both A and B, and presence of only B. Additionally, the character "/" herein generally indicates an "or" relationship between the associated objects.

In the description of the embodiments of this application, the term "multiple" refers to two or more (including two), similarly, "multiple groups" refers to two or more groups (including two groups), and "multiple pieces" refers to two or more pieces (including two pieces). "At least one" refers to one or more (including one, two, three, and the like).

In the description of the embodiments of this application, the orientation or positional relationships indicated by technical terms such as "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial", "circumferential", and the like are based on the orientation or positional relationships shown in the drawings. They are used merely for the convenience of describing the embodiments of this application and for simplifying the description, and do not indicate or imply that the referred device or element must have a specific orientation, be constructed, and operate in a specific orientation, and thus should not be construed as limiting the embodiments of this application.

In the description of the embodiments of this application, unless otherwise explicitly specified and limited, technical terms such as "install", "connect", "connection", "fix", and the like should be understood in a broad sense. For example, it may refer to a fixed connection, a detachable connection, or an integral formation; it may be a mechanical connection or an electrical connection; it may be a direct connection or an indirect connection through an intermediary, or it may be the internal communication or interaction between two elements. For those of ordinary skill in the art, the specific meanings of the above terms in the embodiments of this application can be understood based on specific circumstances.

With the increasing depletion of traditional energy resources, the development of new energy sources has gained significant attention. Due to the modern society's new demand for green energy, optoelectronic devices such as solar cells, which convert light energy into electrical energy through the photoelectric effect, have developed rapidly. Currently, solar cells have advanced to the third generation, namely perovskite solar cells (PSCs). Perovskite solar cells offer advantages such as high photoelectric conversion efficiency and low power generation costs, and they have the potential for integration with buildings, potentially serving as substitutes for curtain wall decorations in high-rise buildings, thereby achieving both lighting and power generation.

Perovskite solar cells are solar cells that utilize perovskite-type organometallic halide semiconductors as light-absorbing materials. As an artificially synthesized material, perovskite has shone brightly in the field of photovoltaic power generation due to its excellent performance, low cost, and significant commercial value. The perovskite layer, as the light-absorbing layer in perovskite solar cells, directly affects the conversion efficiency and stability of the device.

Due to the influence of crystal growth and subsequent processing, perovskite is prone to surface defects. For example, perovskite grain boundaries primarily form two types of defects: (1) undercoordinated cations, such as divalent cations, caused by iodide vacancies; (2) undercoordinated anions, such as halide ions, caused by cation vacancies resulting in uncoordinated anions at the boundaries. Surface defect states formed in perovskite crystals readily lead to non-radiative recombination of charge carriers, thereby affecting their photoelectric conversion efficiency. Currently, molecular modification can be used to passivate perovskite surface defects, but typically, molecules with singular properties, such as Lewis base molecules, are used for modification. For instance, it has been reported that 1-benzyl-3-hydroxypyridinium chloride (1B3HPC) is used to modify perovskite. As a Lewis base, 1B3HPC can passivate undercoordinated divalent cations (such as Pb²⁺) on the perovskite surface, which act as Lewis acids, forming Lewis adducts. However, it cannot passivate undercoordinated halide ions, resulting in limited improvement in solar cell performance.

Based on the above considerations, to simultaneously address multiple defects in perovskite, an embodiment of this application designs an ambipolar molecule that possesses both Lewis acid and Lewis base groups, enabling simultaneous passivation of the aforementioned two types of defects in perovskite, thereby further enhancing perovskite performance. Accordingly, the following technical solutions are proposed.

### Ambipolar molecule

According to a first aspect, an embodiment of this application provides an ambipolar molecule, where a chemical structure general formula of the ambipolar molecule is represented by formula I: 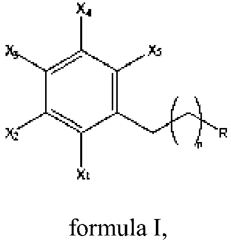 where R includes a Lewis base group, X₁, X₂, X₃, X₄, and X₅ each include at least one of a hydrogen atom and a halogen atom, X₁, X₂, X₃, X₄, and X₅ are not simultaneously hydrogen, and n is an integer greater than or equal to 1.

According to the Lewis acid-base (Lewis acids and bases) theory, also known as the acid-base electron theory: substances (molecules, ions, or atomic groups) that can accept an electron pair are called Lewis acids (Lewis Acid, LA), and substances (molecules, ions, or atomic groups) that can donate an electron pair are called Lewis bases (Lewis Base, LB). Lewis acids are electron pair acceptors, and Lewis bases are electron pair donors.

The ambipolar molecule represented by formula I provided by the embodiments of this application simultaneously possesses a Lewis acid group and a Lewis base group. Specifically, R includes a Lewis base group that can donate an electron pair, while the halogen-substituted benzene ring structure serves as a Lewis acid group that can accept an electron pair. The ambipolar molecule provided by the embodiments of this application can be configured to passivate two types of defects at perovskite grain boundaries, namely undercoordinated anions and undercoordinated cations. Specifically: (1) the halogen-substituted benzene ring structure, as a Lewis acid group, can accept electrons from anions at the perovskite crystal edges to passivate undercoordinated anions in the perovskite crystal, (2) the lone electron pair of the Lewis base at the R end can be provided to undercoordinated cations to passivate anion vacancy defects in the perovskite crystal.

In an embodiment, in the ambipolar molecule represented by formula I provided by the embodiments of this application, -(CH₂)-(CH₂)ₙ- is an intermediate carbon chain connecting the R group and the halogen-substituted benzene ring structure. Specifically, n is 1 to 9, for example, n may be 2, 4, 5, 6, 8, 9, or other values. The distance between the R group and the halogen-substituted benzene ring structure is relatively short, making it less likely to form intermolecular adducts. The ambipolar molecule can flexibly bind with undercoordinated anions and undercoordinated cations as needed, thereby effectively passivating surface defects of perovskite crystals and improving the conversion efficiency and stability of perovskite.

In an embodiment, in the ambipolar molecule represented by formula I, the Lewis base group in R includes at least one of thiophenyl, furanyl, pyridyl, and 3-methylimidazolyl. For example, it may include Lewis base groups with different connection modes such as 2-thiophenyl, 3-thiophenyl, 2-furanyl, 3-furanyl, 2-pyridyl, 3-pyridyl, 4-pyridyl, and the like.

When the ambipolar molecule containing the above types of Lewis base groups is used in perovskite, these Lewis base groups can bind with undercoordinated cations, providing lone electron pairs to the bound cations to passivate anion vacancy defects in the perovskite crystal.

In an embodiment, in the ambipolar molecule represented by formula I, X₁, X₂, X₃, X₄, and X₅ independently include at least one of a hydrogen atom and a halogen atom, and X₁, X₂, X₃, X4, and X5 are not simultaneously hydrogen. It can be understood that the halogen-substituted benzene ring structure may have single substitution, multiple substitutions, or substitutions at different positions. As long as the formed halogen-substituted benzene ring structure possesses the characteristics of a Lewis acid, it falls within the scope of protection of this application. Specifically, the halogen atom may be fluorine, chlorine, bromine, iodine, or the like.

In an embodiment, in the ambipolar molecule represented by formula I, X₁, X₂, X₃, X4, and X5 are a same halogen atom. The fully halogen-substituted benzene ring structure, as a Lewis acid group, can better bind with undercoordinated anions to more effectively passivate undercoordinated anions in the perovskite crystal. In an embodiment, the ambipolar molecule includes the following structure:

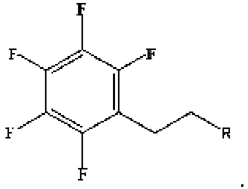

In the ambipolar molecule, the fully fluorine-substituted benzene ring serves as the Lewis acid group, which not only has a stable structure and effectively passivates perovskite grain boundary defects but is also easy to synthesize.

### Preparation method of ambipolar molecule

According to a second aspect, an embodiment of this application provides a preparation method of an ambipolar molecule, including:
subjecting a compound represented by formula II to a hydrogenation reaction to obtain the ambipolar molecule provided by the first aspect of the embodiments of this application:

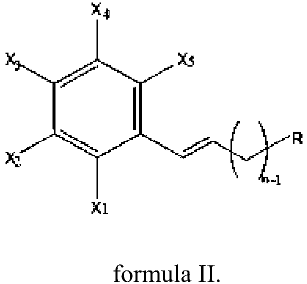

Using the compound represented by formula II for a hydrogenation reaction, thereby connecting the R group and the halogen-substituted benzene ring structure via a saturated carbon chain, yields the ambipolar molecule represented by formula I in the embodiments of this application. This preparation method is not only simple and easy to implement but also produces an ambipolar molecule that, based on Lewis acid and Lewis base groups, effectively passivates surface defects of perovskite crystals, thereby improving the conversion efficiency and stability of perovskite.

In an embodiment, in the raw material compound represented by formula II, R, X₁, X₂, X₃, X4, X5, and n correspond to R, X₁, X₂, X₃, X4, X5, and n in the product ambipolar molecule represented by formula I.

In an embodiment, a temperature of the hydrogenation reaction is 60°C to 80°C (Celsius); exemplarily, the temperature of the addition reaction may be 60°C, 70°C, 75°C, 80°C, or other values. The temperature condition of 60°C to 80°C effectively promotes the hydrogenation reaction.

In an embodiment, a duration of the hydrogenation reaction is 2 h to 8 h (hours); exemplarily, the duration of the addition reaction may be 2 h, 4 h, 5 h, 6 h, 8 h, or other values. The hydrogenation reaction duration of 2 h to 8 h sufficiently achieves the synthesis of the ambipolar molecule.

In an embodiment, the temperature of the hydrogenation reaction is 60°C to 80°C, and the duration of the hydrogenation reaction is 2 h to 8 h. Under the above temperature and duration conditions of the hydrogenation reaction, the compound represented by formula II can fully undergo the hydrogenation reaction to efficiently achieve the synthesis of the ambipolar molecule.

In an embodiment, the hydrogenation reaction is conducted under a condition of a rhodium-based catalyst. The addition of the rhodium-based catalyst increases the rate of the addition reaction, thereby effectively enhancing the synthesis efficiency of the ambipolar molecule represented by formula I as the product. For example, the rhodium-based catalyst may include tris(triphenylphosphine)rhodium(I) chloride.

In an embodiment, the hydrogenation reaction may be conducted in an alcohol-based solvent. Alcohol-based solvents can fully dissolve the raw materials for the hydrogenation reaction. Alcohol-based solvents may include ethanol, propanol, or the like. Taking ethanol as an example, the compound represented by formula II is dissolved in ethanol, purged with nitrogen, and an appropriate amount of the catalyst tris(triphenylphosphine)rhodium(I) chloride is added to maintain a certain hydrogen pressure for the reaction to obtain the product.

In an embodiment, synthesis steps of the compound represented by formula II include: subjecting a compound represented by formula III to a nucleophilic substitution reaction with triethyl phosphite, and then subjecting a product of the nucleophilic substitution reaction to an addition reaction with R-(CH₂)ₙ₋₁-CO: 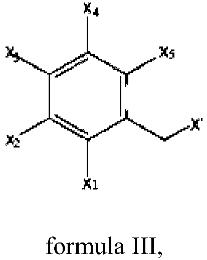 where X' is a halogen atom.

Triethyl phosphite first undergoes a nucleophilic substitution reaction with the halogenated benzene ring hydrocarbon represented by formula III, and the halogen ion causes the cleavage of the ethoxy group to generate a hydrocarbon-substituted diethyl phosphonate derivative. When the β-position of the halogen atom in the halogenated benzene ring hydrocarbon represented by formula III has an electron-withdrawing group, the product generates a phosphonium ylide under the action of a strong base, which then reacts with the aldehyde R-(CH₂)ₙ₋₁-CO to yield the HWE (Horner-Wadsworth-Emmons) reaction product, which is the compound represented by formula II.

Taking the raw material compound represented by formula II, where X₁, X₂, X₃, X4, and X5 are fluorine atoms and n = 1 as an example, the steps of the synthesis method of the compound represented by formula II may include: heating C₆F₅CH₂Br and triethyl phosphite under nitrogen protection for a nucleophilic substitution reaction. The product after vacuum distillation is dissolved in dimethylformamide with sodium methoxide and R-group formaldehyde R-CHO for an addition reaction, followed by purification, drying, and recrystallization to obtain the compound represented by formula II containing a double bond. Subsequently, a hydrogenation reaction is conducted under the condition of a tris(triphenylphosphine)rhodium(I) chloride catalyst to obtain the corresponding ambipolar molecule. The specific process is as follows:

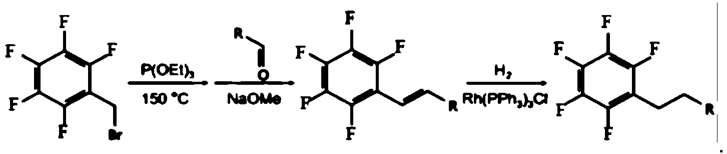

### APPLICATION

According to a third aspect, an embodiment of this application provides an application, namely the application of the ambipolar molecule provided by the first aspect of the embodiments of this application and/or the ambipolar molecule prepared by the preparation method provided by the second aspect of the embodiments of this application as a perovskite crystal passivator.

Perovskite grain boundaries primarily exhibit two types of defects, namely undercoordinated anions and undercoordinated cations, and Lewis acids and Lewis bases can respectively passivate these two types of defects to enhance perovskite performance. The ambipolar molecule represented by formula I designed by the embodiments of this application simultaneously possesses Lewis acid and Lewis base groups, enabling it to passivate undercoordinated anions and undercoordinated cations at perovskite grain boundaries. Additionally, based on the hydrophobic properties of the ambipolar molecule, it can improve the moisture stability of perovskite. Therefore, using the ambipolar molecule as a perovskite crystal passivator in the synthesis of perovskite can passivate surface defects of perovskite crystals, improving the conversion efficiency and stability of perovskite.

Specifically, the types and preparation methods of the ambipolar molecule represented by formula I are as described above.

### Perovskite material

According to a fourth aspect, an embodiment of this application provides a perovskite material, where the perovskite material includes a perovskite crystal and the ambipolar molecule provided by the first aspect of the embodiments of this application and/or the ambipolar molecule prepared by the preparation method provided by the second aspect of the embodiments of this application, bound to the perovskite crystal.

The ambipolar molecule represented by formula I, which simultaneously possesses Lewis acid and Lewis base groups and exhibits hydrophobicity, is used in the perovskite material as a passivator bound to the perovskite crystal, enabling passivation of perovskite grain boundary defects and improving the conversion efficiency and stability of the perovskite material.

In some embodiments, the chemical formula of the perovskite in the embodiments of this application may be ABX₃; where A is a monovalent cation, which may be an organic cation or an inorganic cation, specifically including at least one of CH₃NH₃⁺, Cs⁺, and CH(NH₂)₂⁺; B is a divalent cation, specifically including at least one of Pb²⁺ and Sn²⁺; and X is a monovalent anion, specifically including at least one of Cl⁻, Br⁻, and I⁻.

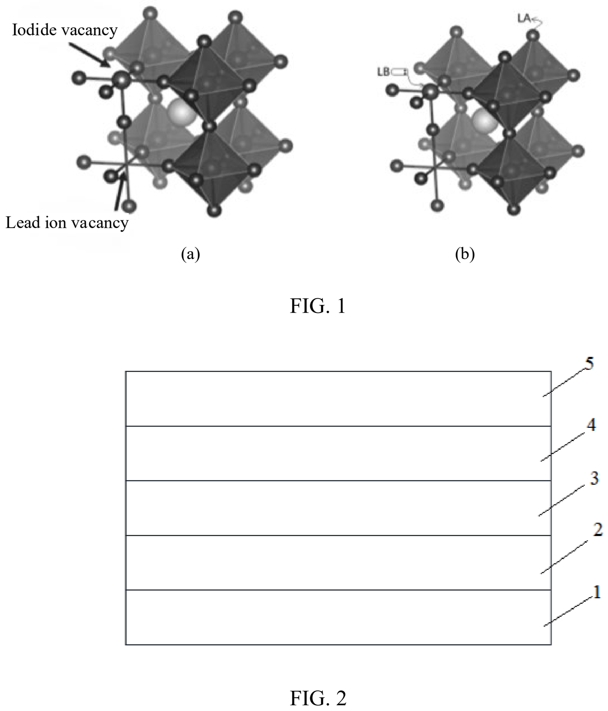

In some embodiments, taking the divalent cation in the perovskite as Pb²⁺ and the monovalent anion as I⁻ as an example, as shown in FIG. 1, before using the ambipolar molecule (a in FIG. 1), the perovskite grain boundaries exhibit two types of defects: undercoordinated iodide ions (that is, presence of lead ion vacancies) and undercoordinated lead ions (that is, presence of iodide vacancies). After using the ambipolar molecule (b in FIG. 1), the Lewis acid (LA) group at one end of some ambipolar molecules can bind with undercoordinated iodide ions, accepting electrons from iodide ions at the perovskite crystal edges to passivate undercoordinated iodide ions in the perovskite crystal; the Lewis base (LB) group at one end of other ambipolar molecules can bind with undercoordinated lead ions, providing lone electron pairs to the bound lead ions to passivate iodide ion vacancy defects in the perovskite crystal.

Based on the formation of adducts by Lewis acids and Lewis bases, the ambipolar molecule provided by the embodiments of this application can act as both a Lewis acid and a Lewis base. The end of the ambipolar molecule with the Lewis acid group flows toward exposed iodide, and the end with the Lewis base can also flow toward exposed undercoordinated lead ions for binding. This effectively passivates both types of grain boundary defects in perovskite, improving the conversion efficiency and stability of the perovskite material.

In an embodiment, a molar ratio of the perovskite crystal to the ambipolar molecule is 1:(0.0001 to 0.002). Exemplarily, the molar ratio of the perovskite crystal ABX₃ to the ambipolar molecule may be 1:0.0001, 1:0.0002, 1:0.0005, 1:0.0006, 1:0.0008, 1:0.001, 1:0.0015, 1:0.002, or other ratios. The ambipolar molecule at the above molar ratios effectively passivates the perovskite crystal.

In an embodiment, the ambipolar molecule represented by formula I can be added as an additive to the perovskite precursor solution solvent, followed by annealing and crystallization to obtain a perovskite material passivated by the ambipolar molecule. Specifically, a perovskite precursor solution of ABX₃ is prepared, then the ambipolar molecule is added and stirred uniformly, annealed at 80°C to 100°C for 20 to 40 minutes, and then cooled to room temperature to obtain a perovskite layer, forming a perovskite material in the form of a film layer. During the annealing and crystallization process, on one hand, the ambipolar molecule slows down the crystallization process of perovskite, thereby increasing the perovskite grain size; on the other hand, the ambipolar molecule with Lewis acid and Lewis base groups has a passivation effect at the perovskite grain boundaries, reducing the defect density of the perovskite layer, thereby enhancing the conversion efficiency and stability of perovskite.

In the above process, the amount of ambipolar molecule added to the perovskite precursor solution may be 0.01 mol% to 0.2 mol% (percent molar ratio relative to perovskite ABX₃); such an amount of ambipolar molecule effectively passivates the perovskite crystal.

### Optoelectronic device

According to a fifth aspect, an embodiment of this application provides an optoelectronic device including a perovskite layer, where the perovskite layer includes the perovskite material provided by the fourth aspect of the embodiments of this application.

Since the perovskite layer of the optoelectronic device provided by the embodiments of this application includes a unique perovskite material, such a perovskite layer has fewer grain boundary defects and good moisture resistance, thereby enabling the optoelectronic device to exhibit excellent photoelectric conversion efficiency and stability.

In an embodiment, the optoelectronic device includes a solar cell. The solar cell uses the unique perovskite material of the embodiments of this application, enabling better conversion of light energy into electrical energy, resulting in excellent photoelectric conversion efficiency and stability.

In an embodiment, the optoelectronic device includes a first electrode, a hole transport layer, a perovskite layer, an electron transport layer, and a second electrode stacked in sequence. The structure of this optoelectronic device facilitates hole and electron transport within the device, improving the carrier transport performance of the device, thereby further enhancing photoelectric conversion efficiency.

In some embodiments, as shown in FIG. 2, the optoelectronic device includes a first electrode 1, a hole transport layer 2, a perovskite layer 3, an electron transport layer 4, and a second electrode 5 stacked in sequence; where the perovskite layer 3 contains the perovskite material of the embodiments of this application, with the perovskite crystal surface modified and passivated by the ambipolar molecule represented by formula I. This perovskite material has large grain sizes and low defect state density, enabling the device to exhibit excellent photoelectric conversion efficiency and stability. Meanwhile, the structure of this optoelectronic device facilitates hole and electron transport within the device, improving the carrier transport performance of the device, thereby further enhancing photoelectric conversion efficiency.

In some embodiments, the first electrode 1 may be a transparent conductive substrate, including but not limited to the following materials: fluorine-doped tin oxide (FTO), indium-doped tin oxide (ITO), aluminum-doped zinc oxide (AZO), boron-doped zinc oxide (BZO), indium-doped zinc oxide (IZO), and the like.

In some embodiments, the hole transport layer 2 may be at least one of the following materials and their derivatives, as well as materials obtained by doping or passivation thereof: poly[bis(4-phenyl)(2,4,6-trimethylphenyl)amine] (PTAA), poly-3-hexylthiophene (P3HT), triphenylene-based triphenylamine (H101), 3,4-ethylenedioxythiophene-methoxytriphenylamine (EDOT-OMeTPA), N-(4-aniline)carbazole-spirobifluorene (CzPAF-SBF), poly(3,4-ethylenedioxythiophene):poly(styrenesulfonate) (PEDOT:PSS), polythiophene, nickel oxide (NiOₓ), molybdenum oxide (MoO₃), copper(I) iodide (CuI), copper(I) oxide (CuO), and the like.

In some embodiments, the electron transport layer 4 may be at least one of the following materials and their derivatives, as well as materials obtained by doping or passivation thereof: [6,6]-phenyl-C61-butyric acid methyl ester (PC61BM), [6,6]-phenyl-C71-butyric acid methyl ester (PC71BM), fullerene C₆₀ (C₆₀), fullerene C₇₀ (C₇₀), tin dioxide (SnO₂), zinc oxide (ZnO), and the like.

In some embodiments, the second electrode 5 may be a metal electrode or one or more types of conductive oxide electrodes.

Taking the optoelectronic device as a perovskite solar cell as an example, the preparation method includes: providing a first electrode 1, preparing a hole transport layer 2 on the first electrode 1, preparing a perovskite layer 3 on the hole transport layer 2 (specifically, preparing a perovskite film using the preparation method of the embodiments of this application), preparing an electron transport layer 4 on the perovskite layer 3, and preparing a second electrode 5 on the electron transport layer 4.

### Electric apparatus

According to a sixth aspect, an embodiment of this application further provides an electric apparatus, where the electric apparatus includes the optoelectronic device provided by the fifth aspect of the embodiments of this application. The optoelectronic device can be used as a photoelectric conversion device in the electric apparatus, converting light energy into electrical energy. Therefore, the electric apparatus of the embodiments of this application exhibits high photoelectric conversion efficiency and good stability, enabling better operation.

The electric apparatus of the embodiments of this application may include, but is not limited to, household lighting and power supply. For example, when the optoelectronic device utilized is a solar cell, the electric apparatus can be installed on a rooftop or in a courtyard, converting solar energy into electrical energy to provide lighting and power supply for households. As another example, the electric apparatus may be a solar power station, where solar cells can be used to build a solar power station, converting solar energy into electrical energy and transmitting it to the grid to provide clean energy for cities.

### Examples

The following describes examples of this application. The examples described below are exemplary and are used only to explain this application, and should not be construed as limiting this application. For examples where specific techniques or conditions are not specified, they are conducted according to the techniques or conditions described in the literature in the field or according to product specifications. The reagents or instruments used are all conventional products commercially available if no manufacturer is indicated.

### 1. Examples of ambipolar molecule and preparation method thereof

### Example A1

An ambipolar molecule, as represented by formula I, where n = 1, R is 2-pyridyl, and X₁, X₂, X₃, X4, and X5 are all fluorine atoms. That is, the ambipolar molecule is C₆F₅-CH₂CH₂-2-pyridine. The preparation method of the ambipolar molecule was as follows:

C₆F₅CH₂Br and triethyl phosphite (molar ratio 1:2) were heated at 150°C for 2 hours under nitrogen protection. The product after vacuum distillation was dissolved in dimethylformamide with 1.5 equivalents of sodium methoxide and 1 equivalent of 2-pyridinecarboxaldehyde. After stirring at 0°C for 15 minutes, it was stirred at room temperature for 90 minutes. After purification, drying, and recrystallization, an intermediate product containing a double bond was obtained. This intermediate product was dissolved in ethanol, purged with nitrogen for 15 minutes, and 5% molar amount of tris(triphenylphosphine)rhodium(I) chloride relative to the intermediate product was added. In a Parr apparatus, the reaction was maintained at 65°C and 50 psi hydrogen pressure for 5 hours. After the reaction, the ethanol solvent was evaporated, and the product was purified to obtain the final product C₆F₅-CH₂CH₂-2-pyridine.

The resulting ambipolar molecule product was characterized by ¹H NMR (DMSO-d6, 300 MHz). ¹H NMR δ 8.55 (d, J = 7.2 Hz, 1H), 7.62 (d, J = 7.3 Hz, 1H), 7.53 (m, J = 7.2 Hz, 2H), 3.61 (t, J = 7.4 Hz, 2H), 2.80 (t, J = 7.3 Hz, 2H).

### Example A2

An ambipolar molecule C₆F₅-CH₂CH₂-3-pyridine, differing mainly from Example A1 in that R was 3-pyridyl, with all other aspects being identical.

The resulting ambipolar molecule product was characterized by ¹H NMR (DMSO-d6, 300 MHz). ¹¹H NMR δ 8.91 (s, 1H), 8.55 (d, J = 7.2 Hz, 1H), 8.01 (d, J = 7.2 Hz, 1H), 7.29 (m, J = 7.4 Hz, 1H), 3.63 (t, J = 7.4 Hz, 2H), 2.77 (t, J = 7.3 Hz, 2H).

### Example A3

An ambipolar molecule C₆F₅-CH₂CH₂-4-pyridine, differing mainly from Example A1 in that R was 4-pyridyl, with all other aspects being identical.

The resulting ambipolar molecule product was characterized by ¹H NMR (DMSO-d6, 300 MHz). ¹H NMR δ 8.41 (d, J = 7.5 Hz, 2H), 7.40 (d, J = 7.3 Hz, 2H), 3.69 (t, J = 7.4 Hz, 2H), 2.89 (t, J = 7.3 Hz, 2H).

### Example A4

An ambipolar molecule C₆F₅-CH₂CH₂-2-furan, differing mainly from Example A1 in that R was 2-furanyl, with all other aspects being identical.

The resulting ambipolar molecule product was characterized by ¹H NMR (DMSO-d6, 300 MHz). ¹H NMR δ 7.68 (d, J = 7.6 Hz, 1H), 6.73 (d, J = 7.3 Hz, 1H), 6.43 (d, J = 7.2 Hz, 1H), 3.56 (t, J = 7.4 Hz, 2H), 2.85 (t, J = 7.3 Hz, 2H).

### Example A5

An ambipolar molecule C₆F₅-CH₂CH₂-3-furan, differing mainly from Example A1 in that R was 3-furanyl, with all other aspects being identical.

The resulting ambipolar molecule product was characterized by ¹H NMR (DMSO-d6, 300 MHz). ¹H NMR δ 8.01 (s, J = 7.2 Hz, 1H), 7.05 (d, J = 7.3 Hz, 1H), 6.61 (d, J = 7.3 Hz, 1H), 3.63 (t, J = 7.4 Hz, 2H), 2.77 (t, J = 7.3 Hz, 2H).

### Example A6

An ambipolar molecule C₆F₅-CH₂CH₂-2-thiophene, differing mainly from Example A1 in that R was 2-thiophenyl, with all other aspects being identical.

The resulting ambipolar molecule product was characterized by ¹H NMR (DMSO-d6, 300 MHz). ¹H NMR δ 7.49 (t, J = 7.7 Hz, 1H), 7.29 (m, J = 7.3 Hz, 1H), 7.00 (t, J = 7.5 Hz, 1H), 3.60 (t, J = 7.4 Hz, 2H), 2.81 (t, J = 7.3 Hz, 2H).

### Example A7

An ambipolar molecule C₆F₅-CH₂CH₂-3-thiophene, differing mainly from Example A1 in that R was 3-thiophenyl, with all other aspects being identical.

The resulting ambipolar molecule product was characterized by ¹H NMR (DMSO-d6, 300 MHz). ¹H NMR δ 7.57 (t, J = 7.6 Hz, 1H), 7.51 (s, J = 7.3 Hz, 1H), 7.02 (d, J = 7.5 Hz, 1H), 3.51 (t, J = 7.4 Hz, 2H), 2.78 (t, J = 7.3 Hz, 2H).

### 2. Examples of perovskite solar cell

### Example B1

A perovskite solar cell including an ITO substrate, a hole transport layer, a perovskite layer, an electron transport layer, and a copper electrode stacked in sequence. The preparation steps were as follows:
Step 1: An ITO conductive glass with dimensions of 2.0 × 2.0 cm was taken, and 0.35 cm of ITO was removed from both ends by laser etching to expose the glass substrate. The etched ITO conductive glass was ultrasonically cleaned multiple times sequentially with water, acetone, and isopropanol. The ITO conductive glass was dried under a nitrogen gun to remove the solvent and was placed in an ultraviolet ozone machine for further cleaning to obtain an ultraviolet ozone-treated ITO substrate.
Step 2: A 2 mg/mL solution of poly[bis(4-phenyl)(2,4,6-trimethylphenyl)amine] (PTAA) was spin-coated at a rate of 5000 rpm/s on the ultraviolet ozone-treated ITO substrate. The coated substrate was annealed at 100°C hot plate for 10 minutes to obtain a hole transport layer with a thickness of 40 nm.
Step 3: A 1.4 M MAPbI₃ precursor solution was prepared, then the ambipolar molecule from Example A1 was added (at 0.05 mol% of MAPbI₃). After thorough stirring, the solution was spin-coated at 3000 rpm/s onto the hole transport layer. The coated layer was annealed at 100°C for 30 minutes and cooled to room temperature to obtain a 650 nm thick MAPbI₃ perovskite layer passivated by the ambipolar molecule.
Step 4: Phenyl-C61-butyric acid methyl ester (PCBM) was spin-coated at 1000 rpm/s on the perovskite layer and annealed at 100°C for 10 minutes. Subsequently, a passivation layer of bathocuproine (BCP) was spin-coated at 5000 rpm/s to obtain the electron transport layer. The thickness of the PCBM layer was 30 nm, and the thickness of the BCP layer was 10 nm.
Step 5: The sample obtained in Step 4 was placed into a vapor deposition machine, and a copper electrode was deposited to 80 nm at a rate of 1 A/s to complete the device fabrication.

### Example B2

A perovskite solar cell, identical to Example B1 except that the ambipolar molecule from Example A2 was added to the precursor solution during the preparation of the perovskite layer.

### Example B3

A perovskite solar cell, identical to Example B1 except that the ambipolar molecule from Example A3 was added to the precursor solution during the preparation of the perovskite layer.

### Example B4

A perovskite solar cell, identical to Example B1 except that the ambipolar molecule from Example A4 was added to the precursor solution during the preparation of the perovskite layer.

### Example B5

A perovskite solar cell, identical to Example B1 except that the ambipolar molecule from Example A5 was added to the precursor solution during the preparation of the perovskite layer.

### Example B6

A perovskite solar cell, identical to Example B1 except that the ambipolar molecule from Example A6 was added to the precursor solution during the preparation of the perovskite layer.

### Example B7

A perovskite solar cell, identical to Example B1 except that the ambipolar molecule from Example A7 was added to the precursor solution during the preparation of the perovskite layer.

### Example B8

A perovskite solar cell, identical to Example B7 except that the addition amount of the ambipolar molecule from Example A7 in the precursor solution during the preparation of the perovskite layer was 0.01 mol%.

### Example B9

A perovskite solar cell, identical to Example B7 except that the addition amount of the ambipolar molecule from Example A7 in the precursor solution during the preparation of the perovskite layer was 0.2 mol%.

### Comparative Example 1

A solar cell, identical to Example B1 except that no ambipolar molecule was added during the preparation of the perovskite layer.

### Comparative Example 2

A solar cell, identical to Example B1 except that 1-benzyl-3-hydroxypyridinium chloride was used instead of the ambipolar molecule from Example A1 during the preparation of the perovskite layer.

### Performance test

The efficiency and light stability of the perovskite solar cells were tested under continuous illumination with standard simulated sunlight (AM 1.5G, 100 mW/cm²), with an effective device area of 0.08 cm².

### a. Photoelectric conversion efficiency test of perovskite solar cell:

Under irradiation with standard simulated sunlight (AM 1.5G, 100 mW/cm²), the solar cell was tested to obtain the I-V curve. Based on the I-V curve and the data feedback from the testing equipment, the short-circuit current Jsc (unit: mA/cm²), open-circuit voltage Voc (unit: V), maximum light output current Jmpp (unit: mA), and maximum light output voltage Vmpp (unit: V) were obtained. The fill factor FF of the cell was calculated using the formula FF = (Jmpp × Vmpp) / (Jsc × Voc), unit: %. The photoelectric conversion efficiency PCE of the cell was calculated using the formula PCE = Jsc × Voc × FF / Pw, unit: %; Pw represents the input power, unit: mW.

### b. Stability test of perovskite solar cell:

The prepared solar cell devices were placed in a dry room on a 65°C hot plate for accelerated testing, with humidity around 5%. After 10 days, the device efficiency was retested, and the ratio of the efficiency after 10 days to the initial efficiency was calculated.

The specific experimental results are shown in Table 1.

**Table 1**

| Example | Open-circuit voltage (V) | Short-circuit current (mA/cm²) | Fill factor (%) | Photoelectric conversion efficiency (%) | Ratio of efficiency after 10 days to initial efficiency in stability test (%) |
|---|---|---|---|---|---|
| Comparative Example 1 | 1.099 | 23.61 | 78.98 | 20.49 | 56.23 |
| Comparative Example 2 | 1.098 | 23.72 | 79.90 | 20.81 | 61.93 |
| Example B1 | 1.098 | 23.82 | 79.84 | 20.88 | 64.59 |
| Example B2 | 1.105 | 24.13 | 80.79 | 21.54 | 63.25 |
| Example B3 | 1.098 | 23.98 | 81.01 | 21.33 | 66.91 |
| Example B4 | 1.096 | 24.02 | 80.23 | 21.12 | 70.34 |
| Example B5 | 1.109 | 24.16 | 82.93 | 22.22 | 70.85 |
| Example B6 | 1.100 | 23.91 | 80.45 | 21.16 | 69.82 |
| Example B7 | 1.109 | 24.15 | 82.64 | 22.13 | 69.34 |
| Example B8 | 1.102 | 23.91 | 80.23 | 21.14 | 67.95 |
| Example B9 | 1.111 | 24.17 | 82.93 | 22.27 | 73.49 |

From the test results in Table 1, it is evident that the perovskite layer of the perovskite solar cell uses the unique ambipolar molecule of the embodiments of this application for passivation. Therefore, compared to the comparative examples, the embodiments of this application not only exhibit better photoelectric conversion efficiency but also demonstrate better efficiency stability in the solar cell devices. Moreover, appropriately increasing the amount of the ambipolar molecule enhances the passivation effect.

Finally, it should be noted that the above embodiments are merely used to illustrate the technical solutions of this application and not to limit them. Although this application has been described in detail with reference to the foregoing embodiments, those of ordinary skill in the art should understand that modifications can still be made to the technical solutions described in the foregoing embodiments, or equivalent substitutions can be made to some or all of the technical features. Such modifications or substitutions do not cause the essence of the corresponding technical solutions to depart from the scope of the technical solutions of the embodiments of this application, and they should be encompassed within the scope of the claims and specification of this application. In particular, as long as there is no structural conflict, the technical features mentioned in each embodiment can be combined in any manner. This application is not limited to the specific embodiments disclosed in this specification but includes all technical solutions falling within the scope of the claims.

## Claims

1. An ambipolar molecule, wherein a chemical structure general formula of the ambipolar molecule is represented by formula I: 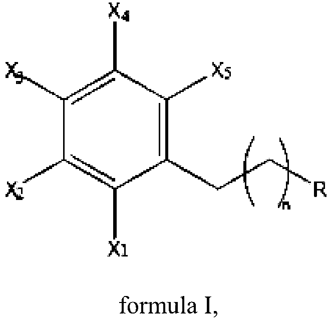 wherein R comprises a Lewis base group, X₁, X₂, X₃, X₄, and X₅ each comprise at least one of a hydrogen atom and a halogen atom, X₁, X₂, X₃, X₄, and X₅ are not simultaneously hydrogen, and n is an integer greater than or equal to 1.

2. The ambipolar molecule according to claim 1, wherein n is 1 to 9.

3. The ambipolar molecule according to claim 1 or 2, wherein the Lewis base group comprises at least one of thiophenyl, furanyl, pyridyl, and 3-methylimidazolyl.

4. The ambipolar molecule according to any one of claims 1 to 3, wherein X₁, X₂, X₃, X₄, and X₅ are a same halogen atom.

5. The ambipolar molecule according to any one of claims 1 to 4, wherein the ambipolar molecule comprises:

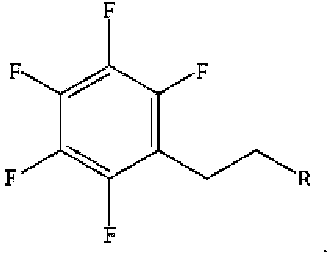

6. A preparation method of an ambipolar molecule, comprising:
subjecting a compound represented by formula II to a hydrogenation reaction to obtain the ambipolar molecule according to any one of claims 1 to 5:

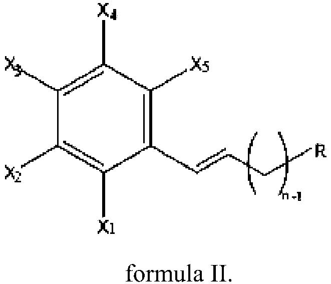

7. The preparation method according to claim 6, wherein a temperature of the hydrogenation reaction is 60°C to 80°C; and/or,
a duration of the hydrogenation reaction is 2 h to 8 h.

8. The preparation method according to claim 6 or 7, wherein the hydrogenation reaction is conducted under a condition of a rhodium-based catalyst; and/or,
the hydrogenation reaction is conducted in an alcohol-based solvent.

9. The preparation method according to any one of claims 6 to 8, wherein synthesis steps of the compound represented by formula II comprise: subjecting a compound represented by formula III to a nucleophilic substitution reaction with triethyl phosphite, and then subjecting a product of the nucleophilic substitution reaction to an addition reaction with R-(CH₂)ₙ₋₁-CO: 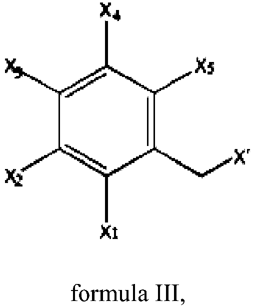 wherein X' is a halogen atom.

10. An application of the ambipolar molecule according to any one of claims 1 to 5 and/or the ambipolar molecule prepared by the preparation method according to any one of claims 6 to 9 as a perovskite crystal passivator.

11. A perovskite material, wherein the perovskite material comprises a perovskite crystal and the ambipolar molecule according to any one of claims 1 to 5 and/or the ambipolar molecule prepared by the preparation method according to any one of claims 6 to 9, bound to the perovskite.

12. The perovskite material according to claim 11, wherein a molar ratio of the perovskite crystal to the ambipolar molecule is 1:(0.0001 to 0.002).

13. An optoelectronic device comprising a perovskite layer, wherein the perovskite layer comprises the perovskite material according to claim 11 or 12.

14. The optoelectronic device according to claim 13, wherein the optoelectronic device comprises a solar cell.

15. An electric apparatus, wherein the electric apparatus comprises the optoelectronic device according to claim 13 or 14.
